(19) Europäisches Patentamt / European Patent Office / Office européen des brevets

(11) **EP 3 462 404 A1**

(12) **DEMANDE DE BREVET EUROPEEN**

(43) Date de publication:
**03.04.2019 Bulletin 2019/14**

(21) Numéro de dépôt: **18185887.9**

(22) Date de dépôt: **26.07.2018**

(51) Int Cl.:
*G06Q 50/30* (2012.01)          *B60W 40/08* (2012.01)
*B60W 40/09* (2012.01)          *B60W 40/12* (2012.01)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **29.09.2017 FR 1759129**

(71) Demandeur: **IFP Energies nouvelles**
**92852 Rueil-Malmaison Cedex (FR)**

(72) Inventeurs:
• **THIBAULT, Laurent**
  **92400 COURBEVOIE (FR)**
• **DEGEILH, Philippe**
  **75012 PARIS (FR)**
• **CORDE, Gilles**
  **91430 IGNY (FR)**

(74) Mandataire: **IFP Energies nouvelles**
**Département Propriété Industrielle**
**Rond Point de l'échangeur de Solaize**
**BP3**
**69360 Solaize (FR)**

(54) **PROCÉDÉ DE DÉTERMINATION D'INDICATEURS SUR LE CARACTÈRE POLLUANT DE LA MOBILITÉ TENANT COMPTE DE L'USAGE RÉEL**

(57) L'invention concerne un procédé de détermination d'indicateurs sur le caractère polluant de la mobilité globale d'un usager comprenant les étapes suivantes : détection des déplacements en modes de transport doux et des déplacements en véhicule motorisé au moyen d'un téléphone intelligent; détermination de l'empreinte environnementale réelle liée aux émissions de polluants pour chaque déplacement en véhicule motorisé au moyen d'un modèle d'estimation des émissions de polluants tenant compte des caractéristiques macroscopiques dudit véhicule et d'un profil de vitesse réel représentant le style de conduite de l'usager ; détermination d'une empreinte environnementale cible à partir de chacun desdits déplacements en véhicule motorisé en prenant en compte un profil de vitesse cible représentant un style de conduite optimal dans ledit modèle d'estimation des émissions de polluants ; décomposition de l'empreinte environnementale de chacun desdits déplacements en véhicule motorisé en une empreinte liée au véhicule, une empreinte liée au style de conduite et une empreinte liée au type de trajet ; détermination de l'empreinte environnementale globale de la mobilité de l'usager prenant en compte l'ensemble des déplacements en modes de transport doux et des déplacements en véhicule motorisé sur une fenêtre temporelle donnée, de préférence la journée ; détermination d'indicateurs d'éco-conduite et/ou d'indicateurs d'impact environnemental de sa mobilité globale pour l'usager.

FIG.1

**Description**

**Domaine technique de l'invention**

La présente invention concerne le domaine de l'éco-conduite, et plus généralement celui de la qualification du caractère polluant de la mobilité globale d'un usager.

**Art antérieur**

A l'heure actuelle, l'amélioration de la qualité de l'air est une priorité pour les villes. Pour y parvenir la solution privilégiée actuellement consiste à interdire la circulation à certains véhicules trop polluants (exemple des vignettes CRIT'AIR). Pourtant, pour un même véhicule et un même trajet, les niveaux d'émissions polluantes peuvent varier significativement en fonction de la manière dont le véhicule est conduit. Cet impact, pourtant réel, demeure relativement méconnu, car pour l'heure difficile à quantifier en dehors de l'utilisation d'une instrumentation de laboratoire très onéreuse.

La demande de brevet FR 3049653, de la Demanderesse, décrit un procédé de détermination des émissions de polluants d'un véhicule donné au moyen de paramètres macroscopiques pour un trajet donné. Cependant ce procédé ne prend en compte que les déplacements en véhicule motorisé, ne tient pas compte du style de conduite de l'usager et ne permet pas de générer de conseils d'éco-conduite.

Un état de l'art détaillé sur les modèles d'estimation des polluants est disponible dans la demande de brevet FR 3 049 653 .

A grande échelle, l'estimation des émissions polluantes consiste à utiliser des coefficients, appelés « Facteurs d'émissions ». L'implémentation la plus connue de cette approche s'appelle COPERT, mais cette approche ne tient pas compte de l'usage réel puisqu'il s'agit de valeurs moyennes pour un véhicule donné, indépendantes de son usage.

En complément il est utile de citer la demande de brevet EP2166309 A2, qui propose un système affichant au conducteur les émissions polluantes instantanées basé sur une cartographie ou fournie directement par le calculateur moteur. Le système décrit dans cette demande ne permet donc pas de calculer le potentiel maximal de gain admissible, ni de générer de conseils d'éco-conduite. De plus, il ne s'agit pas d'une solution qui peut être déployée à grande échelle, puisqu'elle nécessite de mesurer les émissions de chaque véhicule (dans le cas de l'utilisation de la cartographie) ou de se connecter aux calculateurs de chaque véhicule, ce qui est aujourd'hui impossible.

Concernant l'éco-conduite, il n'existe pas dans l'art antérieur d'approche tenant compte de la qualité de l'air. Une approche usuellement retenue consiste à prendre en compte la consommation de carburant et/ou le dioxyde de carbone, comme par exemple dans le demande de brevet FR 2 994 923 de la Demanderesse qui porte sur un procédé de détermination d'un indicateur énergétique d'un déplacement d'un véhicule et la détermination d'une vitesse optimale qui minimise la consommation énergétique.

Le besoin d'outils diffusables à grand échelle pour permettre la responsabilisation des usagers par rapport à leur mobilité et à leur style de conduite reste important dans un objectif d'amélioration de la qualité de l'air.

A cet effet, la présente invention permet de réaliser un système complet permettant à chaque usager de mesurer et d'améliorer l'empreinte polluante de sa mobilité globale, en véhicule motorisé comme en modes de transport doux, au moyen d'une simple application sur téléphone intelligent, appelée ci-après application smartphone, sans nécessiter l'ajout de capteur spécifique. Ce système présente également un bénéfice pour les collectivités en leur permettant de contrôler les niveaux d'émissions en usage réel, sur leur territoire.

**Objet de l'invention**

L'objectif de la présente invention est de compléter les procédés de détermination des émissions polluantes de l'art antérieur à plusieurs niveaux :

- En permettant la détection automatique des trajets et des modes de transport en vue d'un déploiement sur téléphone intelligent ;
- En ajoutant en aval un volet d'éco-conduite pour prendre en compte le comportement du conducteur ;
- En les couplant éventuellement avec d'autres modèles permettant de contrôler et simuler l'efficacité de l'infrastructure routière et des technologies des véhicules.

L'invention permet ainsi de passer d'un simple modèle de détermination des émissions polluantes d'un véhicule donné pour un trajet donné à un système complet d'éco-conduite et de contrôle des polluants ne nécessitant pas d'autres capteurs qu'un GPS. L'avantage principal d'une telle solution est de pouvoir être déployée à grande échelle via une application smartphone. Cette application permet de détecter automatiquement les déplacements (mobilités douces et mobilités en véhicule motorisé), donne des conseils personnalisés et qualifie l'impact "qualité de l'air" du style de conduite en véhicule motorisé grâce à un algorithme calculant la trajectoire de vitesse et les scores polluants idéaux sur un trajet donné. Le procédé selon l'invention permet notamment de déterminer un niveau cible d'émissions polluantes et de donner des conseils au conducteur.

De plus, il n'est pas limité à l'automobile, ou même aux véhicules motorisés, puisqu'il permet de tenir compte de la mobilité au sens large en notant l'utilisation de modes de transports alternatifs (ou modes de transport doux), en affichant des indicateurs d'impact environnemental de la mobilité globale de l'usager et en générant des conseils d'éco-mobilité.

**Résumé de l'invention**

L'invention porte sur un procédé de détermination d'indicateurs sur le caractère polluant de la mobilité globale d'un usager comprenant les étapes suivantes :

a. détection des déplacements en modes de transport doux et des déplacements en véhicule motorisé au moyen d'un téléphone intelligent permettant la mesure de la position et/ou l'altitude et/ou la vitesse de l'usager ;
b. détermination de l'empreinte environnementale réelle liée aux émissions de polluants pour chaque déplacement en véhicule motorisé au moyen d'un modèle d'estimation des émissions de polluants tenant compte des caractéristiques macroscopiques dudit véhicule et d'un profil de vitesse réel représentant le style de conduite de l'usager;
c. détermination d'une empreinte environnementale cible à partir de chacun desdits déplacements en véhicule motorisé en prenant en compte un profil de vitesse cible représentant un style de conduite optimal dans ledit modèle d'estimation des émissions de polluants ;
d. décomposition de l'empreinte environnementale de chacun desdits déplacements en véhicule motorisé en une empreinte liée au véhicule, une empreinte liée au style de conduite et une empreinte liée au type de trajet ;
e. détermination de l'empreinte environnementale globale de la mobilité de l'usager prenant en compte l'ensemble des déplacements en modes de transport doux et des déplacements en véhicule motorisé sur une fenêtre temporelle donnée, de préférence la journée ;
f. détermination d'indicateurs d'éco-conduite et/ou d'indicateurs d'impact environnemental de sa mobilité globale pour l'usager.

La détermination de l'empreinte environnementale dudit déplacement en véhicule motorisé peut être effectuée par agrégation de l'ensemble des émissions polluantes liées aux polluants locaux et globaux dans un seul référentiel, ledit référentiel étant obtenu en réalisant une somme pondérée des émissions de chaque polluant considérés, lesdits coefficients de cette somme pondérée étant choisis en fonction de leur impact sur la santé et l'environnement.

La détermination de l'empreinte environnementale dudit déplacement en véhicule motorisé peut comprendre une détermination des émissions de polluants liées au véhicule motorisé utilisé par l'usager effectuée en acquérant au moins un paramètre macroscopique relatif à la conception dudit véhicule, et en construisant pour ledit véhicule :

i. un modèle dudit véhicule qui relie ladite position et/ou l'altitude et/ou la vitesse dudit véhicule au couple et au régime dudit moteur au moyen d'au moins un paramètre macroscopique ;
ii. un modèle dudit moteur qui relie ledit couple et ledit régime dudit moteur aux émissions de polluants en sortie dudit moteur au moyen d'au moins un paramètre macroscopique ; et
iii. optionnellement un modèle dudit système de post-traitement qui relie lesdites émissions de polluants en sortie dudit moteur au moyen aux émissions de polluants en sortie dudit système de post-traitement au moyen d'au moins un paramètre macroscopique ;

et en réalisant les étapes suivantes :

- on mesure la position, l'altitude et la vitesse dudit véhicule au moyen d'un système de géolocalisation ou d'un téléphone portable ;
- on détermine ledit couple (Cme) et ledit régime (Ne) dudit moteur au moyen dudit modèle de véhicule et desdites mesures ;
- on détermine les émissions de polluants en sortie dudit moteur au moyen dudit modèle du moteur et dudit couple (Cme) et dudit régime (Ne) dudit moteur; et
- éventuellement on détermine les émissions de polluants du véhicule au moyen dudit modèle du système de post-traitement et desdites émissions de polluants en sortie dudit moteur.

De préférence, la différence entre l'empreinte environnementale réelle et l'empreinte environnementale cible permet de déterminer l'empreinte environnementale liée au style de conduite de l'usager pour chacun desdits déplacements.

Avantageusement, la différence entre l'empreinte environnementale réelle et l'empreinte environnementale cible permet de déterminer un indicateur de la qualité du style de conduite de l'usager sous forme d'une note de conduite.

De manière préférée, on détermine un potentiel d'amélioration de l'usager sur ledit déplacement.

Les polluants peuvent être choisis parmi le dioxyde de carbone, les gaz à effet de serre, les oxydes d'azote, les particules, les monoxydes de carbone et/ou les hydrocarbures non brûlés.

Dans un mode de réalisation, le procédé peut comprendre une étape d'agrégation des émissions polluantes de différents usagers ou de passages multiples d'un même usager sur un segment de route faisant partie d'un desdits déplacements en véhicule motorisé afin d'estimer les distributions statistiques et les niveaux moyens d'émissions polluantes sur ledit segment de route.

La détermination de l'empreinte environnementale dudit déplacement en véhicule motorisé peut comprendre une simulation de différentes technologies moteur pour évaluation en usage réel.

L'invention concerne également un produit programme d'ordinateur téléchargeable depuis un réseau de communication et/ou enregistré sur un support lisible par ordinateur et/ou exécutable par un processeur ou un serveur, comprenant des instructions de code de programme pour la mise en oeuvre du procédé tel que décrit ci-dessus, lorsque ledit programme est exécuté sur un ordinateur ou sur un téléphone portable.

L'invention concerne enfin l'utilisation dudit procédé pour estimer l'efficacité écologique d'une infrastructure routière ou d'une réglementation de la circulation routière.

L'invention concerne également l'utilisation dudit procédé pour apporter un retour d'information à l'usager sur l'impact relatif de chaque mode de transport choisi dans l'impact environnemental de sa mobilité globale, de préférence pour générer des conseils d'amélioration écologique de la mobilité globale de l'usager.

## Présentation succincte des figures

D'autres caractéristiques et avantages du procédé selon l'invention, apparaîtront à la lecture de la description ci-après d'exemples non limitatifs de réalisations, en se référant aux figures annexées et décrites ci-après.

La figure 1 illustre l'architecture procédé de détermination d'indicateurs d'éco-conduite et d'éco-mobilité.

La figure 2 illustre l'exemple 1 et représente le potentiel d'amélioration des émissions polluantes en NOx (FIG.2C : instantanée, FIG. 2E : cumulée) et de la consommation (FIG.2B : instantanée, FIG.2D : cumulée) pour un profil de vitesse réel, en relation avec un profit de vitesse cible, les profils étant représentés sur la figure 2A, pour un trajet et un véhicule donné.

La figure 3 illustre l'exemple 2 et représente la décomposition des facteurs impactant les émissions polluantes lors d'une étude réalisée sur des roulages réels effectués pour un même trajet et un même véhicule motorisé et différents conducteurs.

## Description détaillée de l'invention

L'invention permet de réaliser un outil de contrôle et de gestion de l'impact de la mobilité globale d'un utilisateur sur la qualité de l'air. Le procédé selon l'invention permet, à partir d'une méthode d'estimation des émissions polluantes, de construire une solution complète qui peut être déployée sur un smartphone ou à partir de tout autre système muni d'un capteur de géolocalisation.

Le procédé selon l'invention permet de détecter la mobilité de l'usager pour alimenter automatiquement les modèles d'estimation d'émissions de polluants et d'autre part de donner des indicateurs et des conseils d'éco-conduite et/ou d'éco-mobilité à partir des estimations de polluants.

La première étape du procédé assure la détection automatique de la mobilité de l'usager lorsqu'il se déplace sans entrainer une consommation batterie prohibitive, en utilisant au mieux les différents capteurs physiques présents sur le téléphone (GNSS, GSM et centrale inertielle). Cette étape de détection de la mobilité est indispensable pour alimenter les modèles permettant l'estimation des émissions de polluants.

Un volet d'éco-conduite permet aux conducteurs de connaître la part de leurs émissions liée à leur style de conduite et de les aider à la réduire.

La solution complète permet de déterminer des indicateurs qualifiant le caractère polluant de la mobilité d'un utilisateur en tenant compte de ses déplacements réels, dans sa globalité :

- En tenant compte des modes de transports choisis (voiture, vélo, marche, train,...) afin de lui apporter un retour d'information sur l'impact environnemental relatif de chaque mode de transport ;
- En tenant compte de son style de conduite lorsqu'il s'agit de déplacements en voiture et en lui donnant sur chaque trajet la part des émissions qu'il aurait pu réduire en ajustant sa conduite, qui constitue son potentiel d'amélioration.

Le procédé selon l'invention permet également de chiffrer sur une fenêtre temporelle donnée, par exemple chaque jour, l'empreinte polluante globale de la mobilité d'un usager et de projeter ces niveaux d'émissions sur une cartographie.

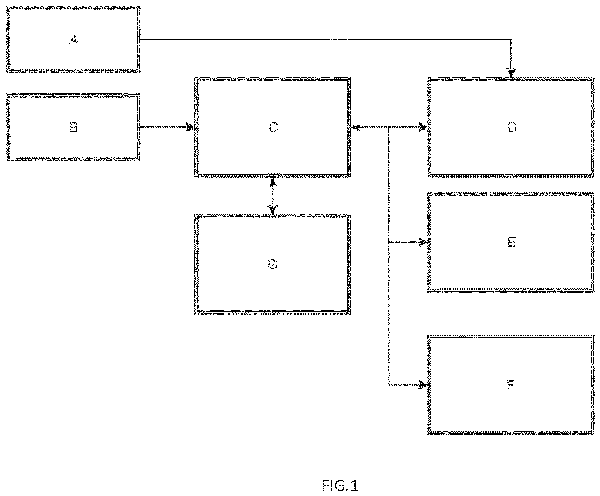

La figure 1 illustre le procédé selon l'invention en présentant l'architecture des différentes étapes de la méthode de détermination des indicateurs sur le caractère polluant de la conduite et de la mobilité globale :

A. Détection des modes de transport doux

B. Détection des déplacements en véhicule motorisé

C. Détermination de l'empreinte environnementale du déplacement en véhicule motorisé tenant compte du style de conduite

D. Détermination de l'empreinte environnementale globale de la mobilité de l'usager sur une fenêtre temporelle donnée, par exemple sur une journée, prenant en compte l'empreinte des mobilités douces et l'empreinte liée aux déplacements en véhicule motorisé

E. Dans le cas d'un trajet en véhicule motorisé, décomposition de l'empreinte environnementale du trajet et détermination d'indicateurs d'éco-conduite pour l'usager.

F. Optionnellement agrégation des émissions polluantes estimées sur chaque segment de route

G. Optionnellement simulation de différentes technologies moteur dans le détermination de l'empreinte environnementale du déplacement en véhicule motorisé pour évaluation en usage réel.

Ces différentes étapes sont détaillées ci-après.

*A. Détection des modes de transport doux*

Les mobilités douces, i.e. non émettrices, doivent être prises en compte dans l'évaluation de l'empreinte environnementale de la mobilité, car elles peuvent être des choix de substitution à des solutions de mobilités polluantes. Les modes de transport doux doivent donc à ce titre être reconnus et encouragés. Le procédé utilisé permet la détection automatique de la marche, de la course et du vélo, grâce à un téléphone intelligent ou à tout autre objet connecté, par exemple une montre connectée, et ce en limitant au maximum la consommation de la batterie. Le procédé permet ainsi un contrôle de la mobilité des utilisateurs du système en nécessitant une intervention minimale de leur part.

Grâce à des moyens de détection simples, utilisant par exemple un accéléromètre, un magnétomètre, un gyroscope, le déplacement est détecté automatiquement, et le mode de transport identifié. Les données de sortie obtenues à l'issue de cette étape de détection sont le mode de transport, l'heure de départ, la durée du trajet et la distance parcourue.

*B. Détection des déplacements en véhicule motorisé*

Le procédé selon l'invention vise à détecter de manière automatique via un smartphone, ou tout autre objet connecté, comme une montre connectée, les déplacements en véhicules motorisés, en maximisant la sensibilité de détection du début de chaque trajet et la précision des données recueillies, tout en limitant la consommation de batterie de la fonction. Cette fonction ne nécessite pas l'utilisation permanente de la géolocalisation (GNSS). Elle se base sur l'observation des changements d'antenne GSM, et/ou sur l'identification du type d'activité de l'utilisateur, détaillé dans le bloc précédent. Lorsqu'un changement d'antenne est détecté ou que l'activité actuellement détectée est un déplacement en véhicule motorisé, voiture par exemple, le capteur GNSS du smartphone est activé pendant un temps donné. Si les mesures de vitesses enregistrées correspondent bien à une vitesse représentative d'un déplacement en véhicule motorisé (cas d'utilisation de véhicule motorisé) alors l'enregistrement d'un nouveau déplacement en véhicule motorisé est lancé et le GNSS reste activé jusqu'à la fin de celui-ci. Au cours du trajet, les signaux de vitesse et d'altitude sont enregistrés à chaque instant, ce qui permet de tenir compte du style de conduite de manière intrinsèque. La fin du trajet est déterminée par une condition sur la vitesse. En fin de trajet, les données enregistrées sont envoyées sur un serveur ou stockées en local pour un envoi ultérieur (au cas notamment où il y ait une absence de réseau en fin de trajet). L'identification du véhicule motorisé utilisé sur chaque déplacement peut être réalisée soit de manière déclarative par l'utilisateur soit de manière automatisée en comparant la route suivie avec certaines routes connues (tramway, bus, aéroglisseur...).

Une correspondance avec une carte routière, dite« mapmatching », qui consiste à projeter les points de la trace GPS (latitude et longitude) sur une carte et via un algorithme d'optimisation à les replacer si besoin sur la route la plus proche est utilisé pour :

- corriger les coordonnées enregistrées en repositionnant les positions sur les routes
- identifier certains types de déplacement en véhicule motorisé, en particuliers les bus de ville.

La fusion des trajets est le procédé d'agrégation de plusieurs trajets enregistrés sur un intervalle de temps donné et qui doivent être considérés comme un seul et même trajet.

*C. Détermination de l'empreinte environnementale du déplacement en tenant compte du style de conduite*

Les émissions des différents polluants générés sur chaque déplacement en véhicule motorisé sont agrégées dans un référentiel unique afin de fournir un indicateur intelligible et facile à suivre dans le temps. Pour chaque trajet *j* réalisé à l'aide d'un véhicule *k,* on détermine une valeur *POPS_{j,k}* en réalisant la somme pondérée des émissions cumulées de chaque polluant i. Les différents polluants sont pris en compte proportionnellement à leur dangerosité. Les facteurs de pondération $\alpha_i$ des différents polluants peuvent être calculés à l'aide de la méthodologie des coûts externes (état de l'art de l'impact sociétal). Ces facteurs sont l'image de la dangerosité de chaque polluant et sont indépendants du véhicule.

$$POPS_{j,k} = \sum_i \alpha_i \times \phi_{i,j,k}^{[\frac{g}{km}]}$$

Une liste non exhaustive des polluants considérés est donnée ci-dessous :

- dioxyde de carbone, gaz à effet de serre
- oxydes d'azote,
- monoxyde de carbone
- particules fines
- hydrocarbures imbrûlés
- dioxyde de soufre

Dans le cas d'un transport partagé (transport en commun ou covoiturage), une pondération peut être appliquée pour normaliser les émissions par le nombre d'usagers.

Les émissions $\phi_{i,j,k}^{[\frac{g}{km}]}$ cumulées de chaque polluant *i* sur le trajet *j* avec le véhicule *k* sont calculées en intégrant les émissions instantanées sur le trajet $\psi_{i,j,k}^{[\frac{g}{h}]}(t),$ elle-même calculées à l'aide de modèles de polluant connus de l'art antérieur, tel que par exemple détaillé dans la demande de brevet FR 3049653 FR. D'un point de vue macroscopique, ce calcul peut être exprimé par les équations suivantes :

$$\begin{cases} \phi_{i,j,k}^{[\frac{g}{km}]} = \int_{t_i}^{t_f} \psi_{i,j,k}^{[\frac{g}{h}]}(t)\, dt \\ \psi_{i,j,k}^{[\frac{g}{h}]}(t) = f_k(V_j(t), Alt_j(t), T_j(t), spec_k) \end{cases}$$

avec :

- $V_j(t)$ la vitesse véhicule et *Alt_j(t)* les profils de vitesse et d'altitude mesurés par le GNSS sur le trajet *j*
- $T_j(t)$ la température extérieure, recalculée à partir des coordonnées GNSS
- $f_k$ le modèle retenu pour le véhicule *k* et *spec_k* les spécifications technologiques du véhicule utilisées pour paramétrer les modèles sont par exemple :

  - le type de motorisation (essence, Diesel,..)
  - le niveau de norme d'homologation (Euro 1, Euro 2, ...)
  - la cylindrée
  - le couple maximal et le régime moteur associé
  - la puissance maximale et le régime moteur associé
  - la masse du véhicule
  - le type de boîte de véhicule
  - le type de système de post traitement

- le type de système d'injection
- le niveau d'hybridation

*D. Détermination de l'empreinte environnementale globale de la mobilité sur une fenêtre temporelle donnée, par exemple la journée*

L'ensemble des empreintes polluants générés par chacun des déplacements pendant une fenêtre temporelle donnée, par exemple au cours de la journée (quel qu'en soit le mode) sont agrégées pour fournir un indicateur unique sur ladite fenêtre temporelle. Cette agrégation est faite en calculant une moyenne pondérée par les distances parcourues de chaque trajet $d_j^{[km]}$ à l'aide de la formule suivante, par exemple pour une journée :

$$POPS_{jour} = \frac{\sum_j d_j^{[km]} * POPS_{j,k}}{\sum_j d_j^{[km]}}$$

Cette détermination de l'empreinte environnementale globale de la mobilité_permet d'estimer l'impact environnemental de la mobilité globale de l'usager. Cette détermination peut également permettre de générer des conseils d'éco-mobilité, en mettant en évidence les déplacements responsables de hauts niveaux d'émission et de donner des conseils applicables au conducteur pour les éliminer ou les réduire. L'objectif est de favoriser les modes de transport non émissifs, en donnant à l'usager un potentiel d'amélioration sur sa mobilité globale. Par exemple sur un trajet très court ou sur un trajet empruntant une ligne de bus, un conseil peut par ailleurs être donné à l'usager en lui indiquant le gain qui aurait pu être obtenu s'il avait pris un transport en commun ou utilisé un mode de transport non émissif comme la marche ou le vélo.

*E. Décomposition de l'empreinte environnementale du déplacement et déterminations d'indicateurs d'éco-conduite pour l'usager*

En raison des règles de calibration des stratégies de contrôle moteur et des phénomènes physiques en jeu, le niveau des émissions polluantes est très sensible, et ce bien plus que la consommation, aux conditions d'utilisation : style de conduite et type de trajet.

En usage réel, les émissions d'un même véhicule peuvent varier significativement et le présent procédé vise notamment à décomposer l'empreinte globale $POPS_{j,k}$ en trois parts, liées au véhicule, au style de conduite et au type de trajet :

$$POPS_{j,k} = POPS_{veh}(k) + POPS_{drive}(j,k) + POPS_{route}(j,k)$$

**Empreinte liée au véhicule $POPS_{veh}(k)$**

L'empreinte environnementale d'un véhicule est étroitement liée aux technologies mises en oeuvre par celui-ci (type de carburant, type de système de dépollution...) et au niveau de norme auquel il répond. Sur un trajet de référence très faiblement émetteur et avec un style de conduite nominal, l'empreinte environnementale représente le minimum d'émissions d'un véhicule sur un usage idéal. Pour chaque polluant i, l'empreinte véhicule exprimée en gramme par kilomètre est déterminée à l'aide d'un calcul d'émissions polluantes comme décrit précédemment en tenant compte de la vitesse $V_{cycle\ ref}$ et de l'altitude $Alt_{cycle\ ref}$ d'un roulage réel de référence correspondant à un trajet et un style de conduite faiblement émetteur.

$$\begin{cases} POPS_{veh}(k) = \sum_i \alpha_i \times \phi_{i,cycle\,ref,k}^{\left[\frac{g}{km}\right]} \\ \phi_{i,cycle\,ref,k}^{\left[\frac{g}{km}\right]} = \int_{t_i}^{t_f} \psi_{i,cycle\,ref,k}^{\left[\frac{g}{h}\right]}(t)\, dt \\ \psi_{i,cycle\,ref,k}^{\left[\frac{g}{h}\right]}(t) = f_k(V_{cycle\,ref}(t), Alt_{cycle\,ref}(t), T_{cycle\,ref}(t), spec_k) \end{cases}$$

**Empreinte liée au style de conduite $POPS_{drive}(j, k)$**

L'objectif du procédé décrit ci-dessous est d'évaluer la qualité du style de conduite, en déterminant l'empreinte environnementale cible sur le trajet étudié et donc le potentiel d'amélioration. Comme détaillé par la suite, l'objectif est de mettre en évidence les comportements de conduite responsables de hauts niveaux d'émission et de donner des conseils applicables au conducteur pour les éliminer. L'approche se base sur la vitesse instantanée mesurée et sur les caractéristiques du véhicule.

$$V_{cycle\,cible}(t) = f_j(V_{cycle\,reel}(t), spec_k)$$

A partir du profil de vitesse réalisé et des spécificités techniques du véhicule, le procédé $f_j$ détermine un profil de vitesse cible en :

- appliquant des seuils maximaux d'accélération : ces seuils peuvent être constants ou dépendants de la vitesse et dépendre des caractéristiques du véhicules ;
- déterminant une vitesse maximale limite ;
- améliorant le maintien de vitesse et l'anticipation des freinages par le filtrage du profil de vitesse.

Le processus vise à ce que les modifications appliquées au profil de vitesse soient intelligibles par le conducteur pour être convertibles en conseils pratiques directement applicables par celui-ci.
Chacun des profils de vitesse réel et cible sont utilisés comme entrée du même modèle d'émissions polluantes $f_k$ retenu pour le véhicule k, afin de déterminer respectivement les empreintes polluantes réalisée et cible :

- empreinte réalisée sur le trajet :

$$\begin{cases} POPS_{cycle\,reel}(j,k) = \sum_i \alpha_i \times \phi_{i,cycle\,reel,k}^{\left[\frac{g}{km}\right]} \\ \phi_{i,cycle\,reel,k}^{\left[\frac{g}{km}\right]} = \int_{t_i}^{t_f} \psi_{i,cycle\,reel,k}^{\left[\frac{g}{h}\right]}(t)\, dt \\ \psi_{i,cycle\,reel,k}^{\left[\frac{g}{h}\right]}(t) = f_k(V_{cycle\,reel}(t), Alt_{cycle\,reel}(t), T_{cycle\,reel}(t), spec_k) \end{cases}$$

- empreinte cible sur le trajet :

$$\begin{cases} POPS_{cycle\,cible}(j,k) = \sum_i \alpha_i \times \phi_{i,cycle\,cible,k}^{\left[\frac{g}{km}\right]} \\ \phi_{i,cycle\,cible,k}^{\left[\frac{g}{km}\right]} = \int_{t_i}^{t_f} \psi_{i,cycle\,cible,k}^{\left[\frac{g}{h}\right]}(t)\, dt \\ \psi_{i,cycle\,cible,k}^{\left[\frac{g}{h}\right]}(t) = f_k(V_{cycle\,cible}(t), Alt_{cycle\,cible}(t), T_{cycle\,cible}(t), spec_k) \end{cases}$$

La différence de l'empreinte environnementale réalisée et cible permet de déterminer l'empreinte liée au style de conduite, et donc le potentiel d'amélioration sur ce trajet :

$$POPS_{drive}(j,k) = POPS_{cycle\ reel}(j,k) - POPS_{cycle\ cible}(j,k)$$

La connaissance de cette part de l'empreinte due au style de conduite permet de déterminer un indicateur de la qualité du style de conduite :

$$Note\ de\ conduite = f\ (POPS_{drive}(j,k), POPS_{j,k}, spec_k)$$

**Empreinte liée au trajet $POPS_{route}(j,\ k)$**

L'empreinte environnementale cible sur le trajet représente donc l'empreinte environnementale atteignable avec un style de conduite optimal. L'écart entre cette empreinte et l'empreinte référence du véhicule est alors imputable au type même du trajet réalisé : trajets courts défavorisant l'efficacité des systèmes de dépollution, trajets embouteillés ou autoroutiers très énergivores, dénivelés importants :

$$POPS_{route}(j,k) = POPS_{cycle\ cible}(j,k) - POPS_{veh}(k)$$

La part liée à la route dépend également du type de véhicule, puisque les différentes technologies véhicules ne présentent pas la même sensibilité aux conditions de roulage. Par exemple un véhicule muni d'un système « Stop&Start » sera moins pénalisé par une forte congestion.

*F. Agrégation des émissions polluantes sur chaque segment de route*

Dans le cas où les profils d'émissions polluantes sont géolocalisés (latitude et longitude enregistrés), il est possible de les projeter sur une cartographie et d'agréger les émissions de différents utilisateurs ou de passages multiples d'un même utilisateur. Cela permet d'estimer les distributions statistiques et les niveaux moyens d'émissions polluantes sur chaque segment de route. Il devient par exemple possible de dresser la carte des zones critiques en émissions de polluants, avec pour objectif de donner un retour d'information dynamique sur l'efficacité environnementale de l'infrastructure routière et de la réglementation associée. Il s'agit donc d'une valorisation de la présente invention qui peut bénéficier à la fois au grand public, mais également à des collectivités ou entreprises.

*G. Simulation de différentes technologies moteur pour évaluation en usage réel*

Dans le cas de l'application principale du système de la présente invention, les modèles de polluants qui représentent les technologies du moteur et du véhicule sont paramétrés pour être représentatifs des véhicules réellement utilisés lors des déplacements puisque l'objectif est d'estimer au mieux les émissions en usage réel.
Cependant il existe une application alternative du système dans laquelle les modèles peuvent être configurés pour simuler d'autre véhicules, ou simplement d'autres configurations moteurs, dans le but de réaliser des études de simulation. On peut citer deux exemples :

- Simuler les émissions d'un parc des véhicules donné (différent du parc réel) pour évaluer l'efficacité des politiques de circulation différenciée lors des pics de pollution ou réaliser des projections des niveaux d'émissions dans les années futures. Par rapport aux méthodes actuellement utilisées pour de telles études la présente invention présente l'avantage de tenir compte des conditions de roulage en usage réel (profils de vitesse et de pente enregistrés)
- Simuler les émissions avec des véhicules munis de nouvelles briques technologiques moteur afin d'évaluer l'efficacité en usage réel de nouvelles technologies sur la réduction des émissions de polluants.

**Avantages de l'invention**

Par rapport aux procédés d'estimation des émissions polluantes de l'art antérieur, le procédé selon l'invention présente plusieurs avantages, notamment :

- Le procédé selon l'invention permet de contrôler les émissions de polluants locaux et globaux par le transport automobile, grâce à une solution qui peut être déployée à grande échelle et faible coût via l'utilisation de smartphones.

- Le procédé selon l'invention permet de conseiller le conducteur pour l'aider à réduire son empreinte polluant liée aux déplacements en véhicule motorisé, en améliorant son style de conduite par la détermination de son empreinte environnementale et la décomposition de celle-ci en trois facteurs : voiture, conduite et trajet.

- Le procédé selon l'invention permet de conseiller l'usager pour l'aider à réduire son empreinte polluant en améliorant ses modes de transport.
- Le procédé selon l'invention permet la prise en compte de la mobilité globale réelle de l'usager : style de conduite et mode de transports choisis.
- Le procédé selon l'invention permet l'agrégation dans un référentiel unique des polluants locaux et globaux avec des coefficients représentatifs de l'impact de chaque polluant sur la santé et l'environnement.
- Le procédé rend également possible la détermination de l'empreinte globale de la mobilité de l'utilisateur sur une fenêtre temporelle donnée, par exemple chaque journée en tenant compte de l'ensemble des modes de transports utilisés (doux ou en véhicule motorisé).
- Le procédé permet de quantifier de manière précise le potentiel d'amélioration lié au style de conduite de l'usager pour un déplacement réalisé avec un véhicule donné.
- Le caractère écologique de la conduite est quantifié, non seulement par rapport à la consommation de carburant ou au CO2, mais dans un référentiel global des polluants locaux et globaux.
- Le procédé permet de détecter l'ensemble des déplacements d'un usager et à identifier le mode de transport utilisé sans autre capteur que ceux de son smartphone ou tout autre objet connecté, comme une montre connectée, et ce avec une consommation batterie limitée.
- Le procédé permet d'enregistrer pendant un trajet les données nécessaires à la détermination de l'empreinte environnementale de ce déplacement.

## EXEMPLES

### Exemple 1 :

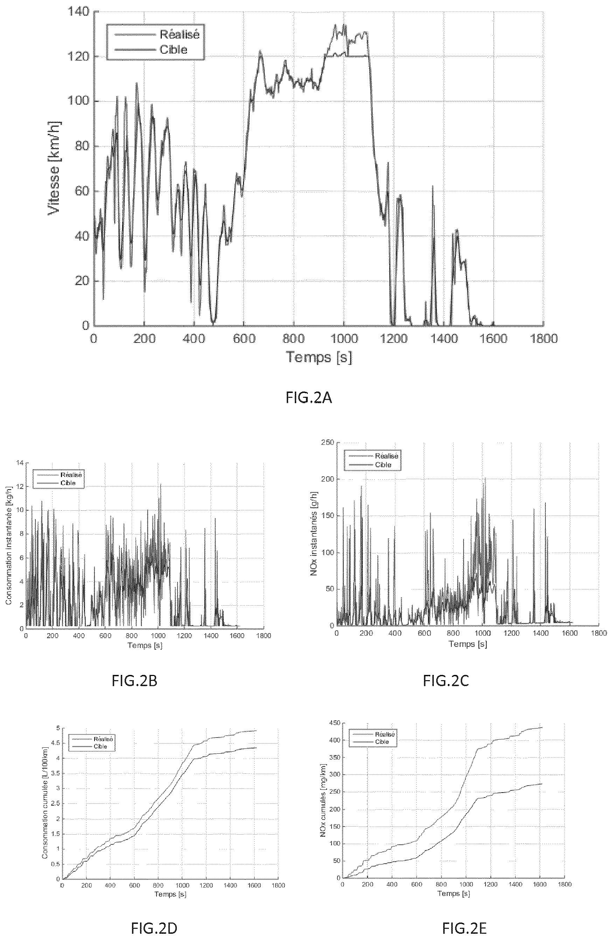

L'exemple 1 ci-dessous illustre un cas réel de décomposition de l'empreinte polluante déterminée pour chaque déplacement en véhicule motorisé, ainsi que la détermination du potentiel d'amélioration. En générant un profil de vitesse cible représentatif d'un style de conduite optimal, le procédé selon l'invention permet de quantifier de manière précise le potentiel d'amélioration lié au style de conduite pour un trajet réalisé avec un véhicule donné. La figure 2 illustre une comparaison entre un profil de vitesse cible généré et un profit de vitesse réel pour un même trajet (2A) et les gains potentiels en consommation de carburant (instantanée en kg/h FIG. 2B, et cumulée en l/100km FIG 2D) et en émissions d'oxydes d'azote (émissions instantanées en g NOx/h FIG. 2C et émissions cumulées en mg NOx/km FIG. 2E) en fonction du temps (en s) pour un véhicule diesel. On observe qu'avec des modifications mineures sur la façon de conduire, il est possible de réduire significativement les émissions polluantes. Ainsi, dans l'exemple ci-dessus, un gain de consommation de 9,8% et une diminution des émissions de NOx de 31,4% pourrait être obtenu si le conducteur s'approchait d'un style de conduite optimal. Ceci constitue le potentiel d'amélioration qui lui est communiqué par le système mettant en oeuvre le procédé selon l'invention, directement sur son téléphone intelligent.

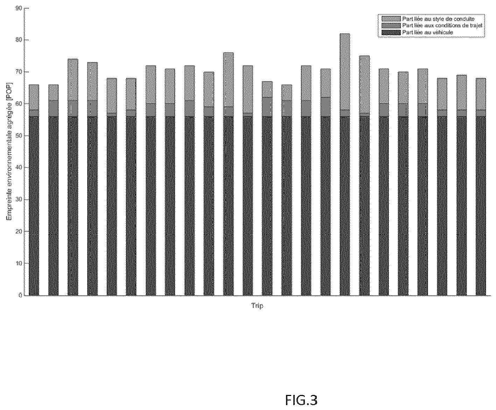

**Exemple** 2 : L'exemple 2 illustre la décomposition des facteurs impactant les émissions polluantes pour 24 utilisateurs différents conduisant un même véhicule sur un même trajet. La figure 3 représente sous forme d'histogrammes l'empreinte environnementale agrégée et sa décomposition en part liée au style de conduite, part liée aux conditions de trajet et part liée au véhicule, en fonction du conducteur. Cette figure illustre le fonctionnement de l'algorithme permettant de déterminer la part des émissions polluantes liée au style de conduite dans l'empreinte polluante globale (empreinte environnementale agrégée POP). En effet, pour un même trajet et un même véhicule, on observe que le niveau de polluant varie sensiblement en fonction du conducteur. Or la difficulté est d'estimer à partir d'un seul passage, s'il existe un potentiel d'amélioration des émissions polluantes lié à l'amélioration du style de conduite. On observe que sur ces différentes répétitions du même trajet :

- la part liée au véhicule ne varie pas, puisque le véhicule reste inchangé
- la part liée au trajet varie peu, puisque l'itinéraire suivi est le même, mais que les conditions de roulages ne sont jamais strictement reproductibles (trafic, feux rouges,...)
- la part liée au style de conduite varie significativement et l'on observe que la somme de la part liée au trajet et de la part liée au véhicule varie très peu, ce qui montre que l'algorithme est bien capable d'estimer avec succès le potentiel de réduction des émissions, donc le potentiel d'amélioration de l'usager en ce qui concerne son style de conduite.

**Revendications**

1. Procédé de détermination d'indicateurs sur le caractère polluant de la mobilité globale d'un usager comprenant les étapes suivantes :

   a. détection des déplacements en modes de transport doux et des déplacements en véhicule motorisé au moyen d'un téléphone intelligent permettant la mesure de la position et/ou l'altitude et/ou la vitesse de l'usager ;
   b. détermination de l'empreinte environnementale réelle liée aux émissions de polluants pour chaque déplacement en véhicule motorisé au moyen d'un modèle d'estimation des émissions de polluants tenant compte des caractéristiques macroscopiques dudit véhicule et d'un profil de vitesse réel représentant le style de conduite de l'usager ;
   c. détermination d'une empreinte environnementale cible à partir de chacun desdits déplacements en véhicule motorisé en prenant en compte un profil de vitesse cible représentant un style de conduite optimal dans ledit modèle d'estimation des émissions de polluants;
   d. décomposition de l'empreinte environnementale de chacun desdits déplacements en véhicule motorisé en une empreinte liée au véhicule, une empreinte liée au style de conduite et une empreinte liée au type de trajet ;
   e. détermination de l'empreinte environnementale globale de la mobilité de l'usager prenant en compte l'ensemble des déplacements en modes de transport doux et des déplacements en véhicule motorisé sur une fenêtre temporelle donnée, de préférence la journée ;
   f. détermination d'indicateurs d'éco-conduite et/ou d'indicateurs d'impact environnemental de sa mobilité globale pour l'usager.

2. Procédé selon la revendication 1 dans lequel la détermination de l'empreinte environnementale dudit déplacement en véhicule motorisé est effectuée par agrégation de l'ensemble des émissions polluantes liées aux polluants locaux et globaux dans un seul référentiel, ledit référentiel étant obtenu en réalisant une somme pondérée des émissions de chaque polluant considérés, lesdits coefficients de cette somme pondérée étant choisis en fonction de leur impact sur la santé et l'environnement.

3. Procédé selon la revendication 1 ou 2 dans lequel la détermination de l'empreinte environnementale dudit déplacement en véhicule motorisé comprend une détermination des émissions de polluants liées au véhicule motorisé utilisé par l'usager effectuée en acquérant au moins un paramètre macroscopique relatif à la conception dudit véhicule, et en construisant pour ledit véhicule :

   i) un modèle dudit véhicule qui relie ladite position et/ou l'altitude et/ou la vitesse dudit véhicule au couple et au régime dudit moteur au moyen d'au moins un paramètre macroscopique ;
   ii) un modèle dudit moteur qui relie ledit couple et ledit régime dudit moteur aux émissions de polluants en sortie dudit moteur au moyen d'au moins un paramètre macroscopique ; et
   iii) optionnellement un modèle dudit système de post-traitement qui relie lesdites émissions de polluants en sortie dudit moteur au moyen aux émissions de polluants en sortie dudit système de post-traitement au moyen d'au moins un paramètre macroscopique ;
   et en réalisant les étapes suivantes :

      a) on mesure la position, l'altitude et la vitesse dudit véhicule au moyen d'un système de géolocalisation ou d'un téléphone portable ;
      b) on détermine ledit couple (Cme) et ledit régime (Ne) dudit moteur au moyen dudit modèle de véhicule et desdites mesures ;
      c) on détermine les émissions de polluants en sortie dudit moteur au moyen dudit modèle du moteur et dudit couple (Cme) et dudit régime (Ne) dudit moteur ; et
      d) éventuellement on détermine les émissions de polluants du véhicule au moyen dudit modèle du système de post-traitement et desdites émissions de polluants en sortie dudit moteur.

4. Procédé selon l'une des revendications précédentes dans lequel la différence entre l'empreinte environnementale réelle et l'empreinte environnementale cible permet de déterminer l'empreinte environnementale liée au style de conduite de l'usager pour chacun desdits déplacements.

5. Procédé selon la revendication 4 dans lequel la différence entre l'empreinte environnementale réelle et l'empreinte environnementale cible permet de déterminer un indicateur de la qualité du style de conduite de l'usager sous forme d'une note de conduite.

**6.** Procédé selon la revendication 4 dans lequel on détermine un potentiel d'amélioration de l'usager sur ledit déplacement.

**7.** Procédé selon l'une des revendications précédentes dans lequel les polluants sont choisis parmi le dioxyde de carbone, les gaz à effet de serre, les oxydes d'azote, les particules, les monoxydes de carbone et/ou les hydrocarbures non brûlés.

**8.** Procédé selon l'une des revendications précédentes comprenant une étape d'agrégation des émissions polluantes de différents usagers ou de passages multiples d'un même usager sur un segment de route faisant partie d'un desdits déplacements en véhicule motorisé afin d'estimer les distributions statistiques et les niveaux moyens d'émissions polluantes sur ledit segment de route.

**9.** Procédé selon l'une des revendications précédentes dans lequel la détermination de l'empreinte environnementale dudit déplacement en véhicule motorisé comprend une simulation de différentes technologies moteur pour évaluation en usage réel.

**10.** Produit programme d'ordinateur téléchargeable depuis un réseau de communication et/ou enregistré sur un support lisible par ordinateur et/ou exécutable par un processeur ou un serveur, comprenant des instructions de code de programme pour la mise en oeuvre du procédé selon l'une des revendications 1 à 9, lorsque ledit programme est exécuté sur un ordinateur ou sur un téléphone portable.

**11.** Utilisation du procédé selon l'une des revendications 1 à 9 pour estimer l'efficacité écologique d'une infrastructure routière ou d'une réglementation de la circulation routière dans laquelle :

- On projette sur une cartographie et on agrège les émissions de différents utilisateurs ou de passages multiples d'un même utilisateur afin d'estimer les distributions statistiques et les niveaux moyens d'émissions polluantes sur chaque segment de route ;
- On dresse la carte des zones critiques en émissions de polluants, pour donner un retour d'information dynamique sur l'efficacité environnementale de l'infrastructure routière et de la réglementation associée.

**12.** Utilisation du procédé selon l'une des revendications 1 à 9 dans laquelle :

- on détermine des indicateurs qualifiant le caractère polluant de la mobilité d'un utilisateur en tenant compte de ses déplacements réels, des modes de transports choisis (voiture, vélo, marche, train,...), de son style de conduite lorsqu'il s'agit de déplacements en voiture pour apporter un retour d'information à l'usager sur l'impact relatif de chaque mode de transport choisi dans l'impact environnemental de sa mobilité globale.

**13.** Utilisation selon la revendication 12 dans laquelle :

- on donne sur chaque trajet à l'utilisateur la part des émissions qu'il aurait pu réduire en ajustant sa conduite, qui constitue son potentiel d'amélioration, pour générer des conseils d'amélioration écologique de la mobilité globale de l'usager.

FIG.1

FIG.2A

FIG.2B

FIG.2C

FIG.2D

FIG.2E

FIG.3

Europäisches
Patentamt

European
Patent Office

Office européen
des brevets

**RAPPORT DE RECHERCHE EUROPEENNE**

Numéro de la demande

EP 18 18 5887

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 2 312 552 A2 (AIRMAX GROUP PLC [GB]) 20 avril 2011 (2011-04-20) * alinéas [0004] - [0005], [0052] - [0054], [0075] - [0079]; figures 14-15 * ----- | 1-13 | INV. G06Q50/30 B60W40/08 B60W40/09 B60W40/12 |
| A | CN 103 786 733 A (UNIV NINGBO) 14 mai 2014 (2014-05-14) * alinéas [0004] - [0009] * ----- | 1-13 | |

DOMAINES TECHNIQUES
RECHERCHES (IPC)

G06Q
B60W

Le présent rapport a été établi pour toutes les revendications

| Lieu de la recherche | Date d'achèvement de la recherche | Examinateur |
|---|---|---|
| Munich | 16 janvier 2019 | Aupiais, Brigitte |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
...............................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 18 18 5887

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-01-2019

| Document brevet cité au rapport de recherche | | Date de publication | Membre(s) de la famille de brevet(s) | | Date de publication |
|---|---|---|---|---|---|
| EP 2312552 | A2 | 20-04-2011 | EP<br>EP<br>GB | 1811481 A1<br>2312552 A2<br>2434346 A | 25-07-2007<br>20-04-2011<br>25-07-2007 |
| CN 103786733 | A | 14-05-2014 | AUCUN | | |

Pour tout renseignement concernant cette annexe : voir Journal Officiel de l'Office européen des brevets, No.12/82

EPO FORM P0460

**EP 3 462 404 A1**

## RÉFÉRENCES CITÉES DANS LA DESCRIPTION

**Documents brevets cités dans la description**

- FR 3049653
- EP 2166309 A2
- FR 2994923